# EUROPÄISCHE PATENTSCHRIFT

(11) **EP 0 915 658 B1**
(45) Veröffentlichungstag und Bekanntmachung des Hinweises auf die Patenterteilung: **16.10.2002**
(21) Anmeldenummer: 97934508.9
(22) Anmeldetag: 21.07.1997
(51) Int. Cl.: A01N 59/12, A01N 47/44, A01N 25/24, A01N 25/10

(54) **FILMBILDENDES MITTEL ZUM SCHUTZ VOR INFEKTIONEN**
FILM-BUILDING AGENT USEFUL TO PROTECT AGAINST INFECTIONS
AGENT FILMOGENE DE PROTECTION CONTRE LES INFECTIONS

(30) Priorität: 30.07.1996 DE 19630622; 24.02.1997 DE 19707081
(43) Veröffentlichungstag der Anmeldung: 19.05.1999
(73) Patentinhaber: Ecolab GmbH & Co. OHG, 40589 Düsseldorf (DE)
(72) Erfinder: BRAGULLA, Siegfried, D-40789 Monheim (DE); BARDONESCHI, Gilbert, F-51300 Bassu (FR); BECKMANN-TOUSSAINT, Jürgen, D-53125 Bonn (DE)
(86) Internationale Anmeldenummer: EP9703922
(87) Internationale Veröffentlichungsnummer: WO98004136

(56) Entgegenhaltungen:
- EP-A- 0 212 866
- WO-A-81/01516
- WO-A-94/23581
- DE-A- 4 225 626
- DE-A- 4 241 079
- US-A- 5 017 369

## Beschreibung

Die Erfindung betrifft ein filmbildendes Mittel zum Schutz vor Infektionen, das insbesondere zum Schutz von Zitzen und Eutern von Kühen, Schafen und Ziegen vor Infektionen nach dem Melken geeignet ist.

Rinder, Schafe und Ziegen werden heute überwiegend durch Anwendung automatischer Melktechniken gemolken. Die durch langjäbrige Züchtungserfolge auf hohe Milchproduktion getrimmten Tiere sind im Bereich der Milchdrüsen und Euter sehr infektionsanfällig. Die Infektionsanfälligkeit wird durch starke Beanspruchung der Zitzen während des Melkens sowie durch ständig auf die Zitzen einwirkende Umgebungsfaktoren wie Wind, Regen, Sonnenstrahlung erhöht.

Durch diese ständige Belastung der Zitzen kommt es bei den Tieren häufig zu Euterentzündungen, die als "subklinische" oder "klinische" Mastitis in der Literatur beschrieben werden. Je nach Stärke der Infektion der erkrankten Euterviertel kommt es zu einer geringen bis drastischen Erhöhung somatischer Zellen in der Milch. Die Erhöhung somatischer Zellen in der Milch über das Maß gesunder Euter (200000 Zellen pro ml) hinaus ist unerwünscht, da sie die Qualität der Milch für die weiteren Verarbeitungsschritte einschränkt oder die Milch im Extremfall ungenießbar werden läßt. Eine drastische Erhöhung somatischer Zellen in der Milch geht oft mit Fieber (klinische Mastitis) einher bis zum Verlust des Tieres durch Notschlachtung.

Eine Ursache für solche Infektionen ist die Einwanderung von euterpathogenen Keimen wie z. B. Pseudomonas aeruginosa, Escherichia coli, Streptococcus uberis, Streptococcus dysagalactiae, Streptococcus agalactiae, Staphylococcus aureus, Enterococcus hirae.

Die größte Gefahr der Euterinfektion besteht unmittelbar nach dem Melkvorgang. In dieser Zeit ist der Strichkanal geöffnet, wodurch es den Keimen aus der Umgebung des Euters möglich ist, in den Strichkanal einzuwandern und Infektionen auszulösen.

Zur Vermeidung bzw. Verringerung des Infektionsrisikos werden die Zitzen der gemolkenen Tiere nach jedem Melkvorgang mit Euter-Dip-Präparaten behandelt. Die Behandlung erfolgt entweder durch Eintauchen oder durch Einsprühen der Zitzen mit der Desinfektionslösung. Durch diese Maßnahme werden die auf der Zitzenhaut vorhandenen Keime innerhalb von 10 Minuten abgetötet, wodurch eine Reduzierung des Infektionsrisikos über den geöffneten Strichkanal erfolgt.

Der Nachteil bei der Anwendung solcher Präparate besteht jedoch darin, daß der Strichkanal bis zu 60 Minuten nach dem Melken geöffnet ist. Die handelsüblichen Zitzen-Präparate sind jedoch derart dünnflüssig, daß sie nur für kurze Zeit (ca. 10 Minuten) über Tropfenbildung an der Zitzenspitze den Strichkanal mechanisch verschließen. Durch Trocknen des Präparates an der Luft oder durch Abreiben des Präparates von der Zitzenspitze beim Hinlegen des Tieres ist dieser Zitzenschutz nach kurzer Zeit wieder aufgehoben. Somit kann wiederum eine Infektion mit euterpathogenen Keimen über den noch geöffneten Strichkanal erfolgen. Ein weiterer Nachteil der bisher verwendeten Produkte ist die sehr hohe Konzentration der Desinfektions-Substanz in den Präparaten, um bei der beschriebenen, kurzen Kontaktzeit eine Desinfektionswirkung zu erreichen

Üblicherweise werden zur Erzielung von ausreichenden Keimabtötungsraten ( 3.0 log-Stufen) an der Zitzenhaut Konzentrationen freien Iods von 2500 bis 10000 ppm verwendet. Derart hohe Iod-Konzentrationen führen zu entsprechender Erhöhung des Iodgehaltes in der Milch, was im Interesse der menschlichen Gesundheit vermieden werden sollte.

Weitere Nachteile solcher Präparate sind die Verwendung von Nonylphenolethoxylaten als Iodträger (Nonoxinol-Iod). Ältere, toxikologische Studien weisen auf die gesundheitlichen Risiken von Nonoxinolen als Rückstand im Lebensmittel hin. In den Studien von Smith und Calandra (Toxicologic Studies of Alkylphenol Polyoxyethylene Surfactants - Toxicology and Applied Pharmacology 14, 315 - 334, 1969) wird die Gefahr von "Myocordnekrosen" beschrieben. Neueste toxikologische Diskussionen betreffen die östrogenen Effekte solcher Substanzen (Bundestagsdrucksache 13/3181-13, Wahlperiode // Environmental Toxicology and Chemistry,. Vol. 15, No. 3, pp. 241 - 248, 1996, Setac // CEFIC Pressestatement "Environmental Estrogen and Endocrine Modulators", 29. April 1996).

Beispielsweise beschreibt die EP-A-90 205 ein Präparat zur Euterpflege und Zitzendesinfektion bei Milchvieh, das als wäßrige Emulsion mit erhöhter Viskosität anwendbar ist und das unter anderem 800 bis 1.200 ppm Iod in Form eines desinfizierend wirkenden Iodkomplexes enthält. Als desinfizierend wirkende Iodkomplexe können Adukte von Iod an Polyglykolether oder an Polyvinylpyrrolidon eingesetzt werden.

Aus der WO 94/23581 sind Zitzendesinfektionsmittel bekannt, die viele der vorstehend genannten Nachteile nicht aufweisen: Sie enthalten eine filmbildende Komponente bestehend aus Polyvinylalkohol, einen polimeren Verdicker sowie einen antimikrobiell wirkenden nichtionischen Iodkomplex. Als nichtionischer Iodkomplex kommen beispielsweise Adukte von Iod an Polyethylenglykol, Polypropylenglykol oder deren Copolymere in Betracht. Als Startmoleküle für die Polymerbildung können Alkoholate, Phenolate und Carboxylate eingesetzt werden.

Gemäß den Ausführungsbeispielen werden Nonylphenolethoxylate als Iodträger eingesetzt.

Die Aufgabe der vorliegenden Erfindung besteht darin, ein filmbildendes Mittel zum Schutz vor Infektionen von Zitzen und Eutern von Milchvieh zur Verfügung zu stellen, das ohne die ökologisch und toxikologisch bedenklichen Nonylphenolethoxylate auskommt. Dabei kommen als toxikologisch vorteilhafte Alternativen einerseits Chlorhexidin und andererseits Iod in Form von Polyvinylpyrrolidon-Iod in Betracht. Versucht man jedoch, in den filmbildenden Mitteln der WO 94/23581 das Nonylphenolethoxylat-Iod durch diese vorteilhafteren Desinfektionswirkstoffe zu ersetzen, so erhält man je nach Fomulierung entweder festhaftende Filme, die sich in einem praxisrelevanten Zeitraum nicht entfernen lassen, oder die Filme bleiben feucht-klebrig, so daß sie beim Liegen der Kühe entweder abgerieben werden oder durch Anhaften von Schmutz zu stark verschmutzten Eutern führen. Daher stellt sich die zusätzliche Aufgabe, ein alternatives filmbildendes Mittel zur Verfügung zu stellen, das gut haftende, trockene, die Zitzenhaut schützende Filme bildet, die sich innerhalb kurzer Zeit, beispielsweise im Bereich von etwa 10 bis etwa 60 Sekunden durch Einwirken von Feuchtigkeit entfernen lassen.

In der US-A-5,017,369 wird ein filmbildendes Mittel zum Schutz von Zitzen und Eutern von Rindern beschrieben. Das wäßrige Mittel enthält 1 bis 16 Gew.-% eines partiell hydrolysierten Polyvinylalkohols mit mindetens 2 Mol-% an restlichen Vinylacetat-Einheiten sowie eine wirksame Menge eines antimikrobiellen Wirkstoffes, beispeilsweise ein Biguanid-Salz, Chlorhexidin oder lodophore.

Demgegenüber betrifft die vorliegende Erfindung ein filmbildendes Mittel zum Schutz vor Infektionen, das eine filmbildende Komponente und einen oder mehrere desinfizierende Wirkstoffe enthält, dadurch gekennzeichnet, daß es
a) als filmbildende Komponente 10 bis 100 g/l Polyvinylalkohol-Polymere oder Copolymere mit Molmassen im Bereich von 18.000 bis 200.000,
b) als desinfizierenden Wirkstoff 0,2 bis 3 g/l lod in Form von Polyvinylpyrrolidon-lod und/oder 0,5 bis 5 g/l Chlorhexidin und
c) 2 bis 50 g/l Ethercarbonsäuren der allgemeinen Formel R-(OC₂H₄)ₙ-OCH₂-COOH enthält, wobei R für einen Alkylrest mit 4 bis 10 C-Atomen und n für eine Zahl im Bereich 2 bis 8 steht.

Überraschenderweise hat sich gezeigt, daß nur durch die Mitverwendung der Ethercarbonsäuren ein filmbildendes Mittel erhalten wird, das die erforderlichen Filmeigenschaften aufweist. Die verwendbaren Ethercarbonsäuren sind beispielsweise aus der DE-A-42 25 626 bekannt, wo ihre Verwendung als desinfizierende Wirkstoffe beschrieben wird. Herstellen lassen sich derartige Ethercarbonsäuren, indem man die entsprechenden Alkanole mit dem Rest R zunächst ethoxyliert und anschließend mit Chloressigsäure umsetzt. Da bei der Ethoxylierung in der Regel keine Reinsubstanzen mit einem definierten Wert für n entstehen, sondern Substanzgemische von Ethoxylierungsprodukten mit unterschiedlichen Ethoxylierungsgraden, liegen die Ethercarbonsäuren in der Regel als entsprechende Mischprodukte vor. Vorzugsweise werden Ethercarbonsäuren eingesetzt, deren Alkylrest R 4 bis 8 C-Atome aufweist. Vorzugsweise enthält das Mittel 5 bis 20 g/l Ethercarbonsäuren.

Als filmbildende Komponente setzt man Polyvinylalkohol-Polymere oder -Copolymere ein. Um günstige Filmbildungseigenschaften zu erhalten, sollten die Molmassen dieser Polymere, die beispielsweise durch Gelpermeationschromatographie bestimmbar sind, im Bereich von 18.000 bis 200.000, vorzugsweise im Bereich von 50.000 bis 150.000 liegen. Vorzugsweise setzt man die filmbildende Komponente in Mengen zwischen 30 und 50 g/l ein.

Zum Einstellen der Viskosität enthält das Präparat vorzugsweise 1.000 bis 10.000 mg/l eines Verdickungsmittels. Vorzugsweise beträgt die Konzentration des Verdickungsmittels 3.000 bis 6.000 mg/l. Als Verdickungsmittel kommen insbesondere solche in Betracht, die als Lebensmittelzusatzstoffe zugelassen sind. Verwendbar sind beispielsweise Xanthan-Gum, Carboxymethylcellulose oder auch Alginate, die einzeln oder im Gemisch miteinander eingesetzt werden können. Verwendet man Mischungen verschiedener Verdickungsmittel, beziehen sich die vorstehend genannten Konzentrationsangaben auf die Gesamtkonzentration.

Als desinfizierenden Wirkstoff enthält das Präparat: Polyvinylpyrrolidon-Iod und/oder Chlorhexidin. Dabei ist die Verwendung von Polyvinylpyrrolidon-Iod besonders bevorzugt. Dieser Wirkstoff ist als Desinfektionsmittel weit verbreitet. Es besteht aus einem Anlagerungskomplex von molekularem Iod, teilweise auch von Triiodidionen, an Polyvinylpyrrolidon. Vorzugsweise setzt man diesen Wirkstoff in einer solchen Menge ein, daß der Iodgehalt des Mittels im Bereich von etwa 0,4 bis etwa 1,5 g/l und insbesondere im Bereich von etwa 0,5 bis etwa 1 g/l liegt.

Das alternativ zu Polyvinylpyrrolidon-Iod oder zusammen mit diesem verwendbare Chlorhexidin ist als desinfizierender Wirkstoff ebenfalls im Stand der Technik bekannt. Vorzugsweise wird es in Form des Glukonats eingesetzt. Dabei wählt man die Konzentration an Chlorhexidin im Bereich von etwa 5 bis etwa 50 g/l, vorzugsweise im Bereich von etwa 10 bis etwa 30 g/l.

Weiterhin kann das Präparat die zur Zitzenhautpflege üblichen Inhaltsstoffe in üblicher Menge enthalten. Beispiele derartiger zusätzlicher Inhaltsstoffe sind Glycerol oder Alantoin.

Die erfindungsgemäßen Mittel sind anwendungsfertig und stellen eine Alternative zu am Markt erhältlichen Mitteln dar, die ebenfalls filmbildende Eigenschanen haben können, bei denen jedoch Nonylphenolethoxylate als Iodträger eingesetzt werden oder bei denen vor der Anwendung aus mehreren Komponenten eine anwendungsfertige Lösung hergestellt werden muß. Die erfindungsgemäße Kombination von Polyvinylalkohol als filmbildender Komponente, Polyvinylpyrrolidon-Iod und/oder Chlorhexidin als desinfizierender Wirkstoff sowie der Ethercarbonsäuren zur Einstellung der Fihnbildungseigenschaften führt zu einem Mittel, das die gestellten Anforderungen voll erfüllt.

Falls erwünscht, kann das erfindungsgemäße Mittel zusätzlich etwa 1. bis etwa 20 g/l eines oder mehrerer Tenside ausgewählt aus anionischen oder nichtionischen Tensiden enthalten. Als anionische Tenside kommen beispielsweise Alkylsulfate oder -sulfonate sowie Alkylbenzolsulfonate, als nichtionische Tenside insbesondere Fettalkoholethoxylate in Betracht. Vorzugsweise liegt die Tensidkonzentration im Bereich von etwa 2 bis etwa 10 g/l.

Schließlich betrifft die Erfindung die Verwendung des vorstehend beschriebenen Präparats zur Herstellung eines filmbildenden Mittels zum Schutz vor Infektionen von Zitzen und Eutern von Rindern, Schafen und/oder Ziegen nach dem Melken und besonders nach mechanischem Melken. Die Verwendung erfolgt beispielsweise derart, daß man die Zitzen oder Euter von Rindern, Schafen oder Ziegen nach dem Melken in das Mittel eintaucht oder sie mit dem Mittel bestreicht oder besprüht. Vorzugsweise wendet man das Mittel derart an, daß man die Zitzen nach dem Melken in das Mittel eintaucht.

Die erfindungsgemäße Verwendung führt dazu, daß das Mittel nach dem Aufbringen auf Euter oder Zitzen einen sichtbaren, mechanisch festen Film bildet. Mit "sichtbar" ist gemeint, daß der Film mit bloßem Auge erkennbar ist. "Mechanisch fest" heißt, daß der Film geschlossen ist und zumindest von glatten Flächen in zusammenhängenden Stücken abziehbar ist. Der Film verschließt zum einen den Strichkanal mechanisch und ermöglicht zum anderen eine lange Einwirkungszeit des desinfizierenden Wirkstoffs. Hierdurch kann die einzusetzende Menge des desinfizierenden Wirkstoffs gegenüber herkömmlichen Mitteln deutlich verringert werden. Dies vermindert die Gefahr einer Kontamination der Milch mit dem desinfizierenden Wirkstoff beim nächsten Melkvorgang. Trotz der verringerten Anwendungskonzentration wird aufgrund der Filmbildung eine ausreichende Desinfektionswirkung gegenüber euterpathogenen Keimen innerhalb von 10 Minuten auf der Zitzenhaut erzielt. Vor dem nächsten Melkvorgang kann der schützende Film innerhalb kurzer Zeit, etwa im Bereich von 10 bis 60 Sekunden, mit Hilfe feuchter Tücher oder unter Verwendung anderer wäßriger Einweichmittel sehr leicht von der Zitzenhaut entfernt werden. Auch hierin liegt ein Vorteil gegenüber den Mitteln der WO 94/23581, wonach das vollständige Entfernen des Films mehr als eine Minute, beispielsweise etwa 5 Minuten, benötigt. Die Erfindung stellt demnach toxikologisch verbesserte Präparate zur Verfügung, die einen sicheren Schutz der Zitzen und Euter von gemolkenen Tieren vor Neuinfektionen bewirkt

### Beispiele

Ein Test auf desinfizierende Wirkung wurde nach der Vorschrift des National Mastitis Council, USA, Protokoll A, durchgeführt. Nach einer Kontaktzeit von 10 Minuten bei 20 °C wurde eine Abtötungsrate um mehr als 3 Zehnerpotenzen ermittelt (Testkeim: Staphylococcus aureus, Abtötungsrate 3,67 dekadische Logarithmusstufen).

| Verwendete Rezeptur (Gew.-%): | |
|---|---|
| vollentsalztes Wasser | 88,3 % |
| Alkylbenzolsulfonsäure | 0,20 % |
| Polyvinylalkohol-Copolymer | 4,00 % |
| Xanthan-Gum | 0,45 % |
| Glycerol | 5,05 % |
| Polyvinylpyrrolidon-Iod (10% Iod) | 1,00 % |
| Caprylethercarbonsäure | 1,0 % |

Die Tabelle 1 zeigt, daß man beim Einsatz von Polyvinylalkohol als Filmbildner und von Polyvinylpyrrolidon-Iod oder von Chlorhexidin als desinfizierender Komponente je nach weiteren Einsatzstoffen entweder schlecht entfernbare Filme oder klebrige Überzüge erhält. Demgegenüber zeigt die Tabelle 2 anhand von erfindungsgemäßen Ausführungsbeispielen, daß erst der Zusatz unterschiedlicher Alkylethercarbonsäuren zu den erwünschten trockenen und festen, jedoch leicht entfernbaren Filmen führt.

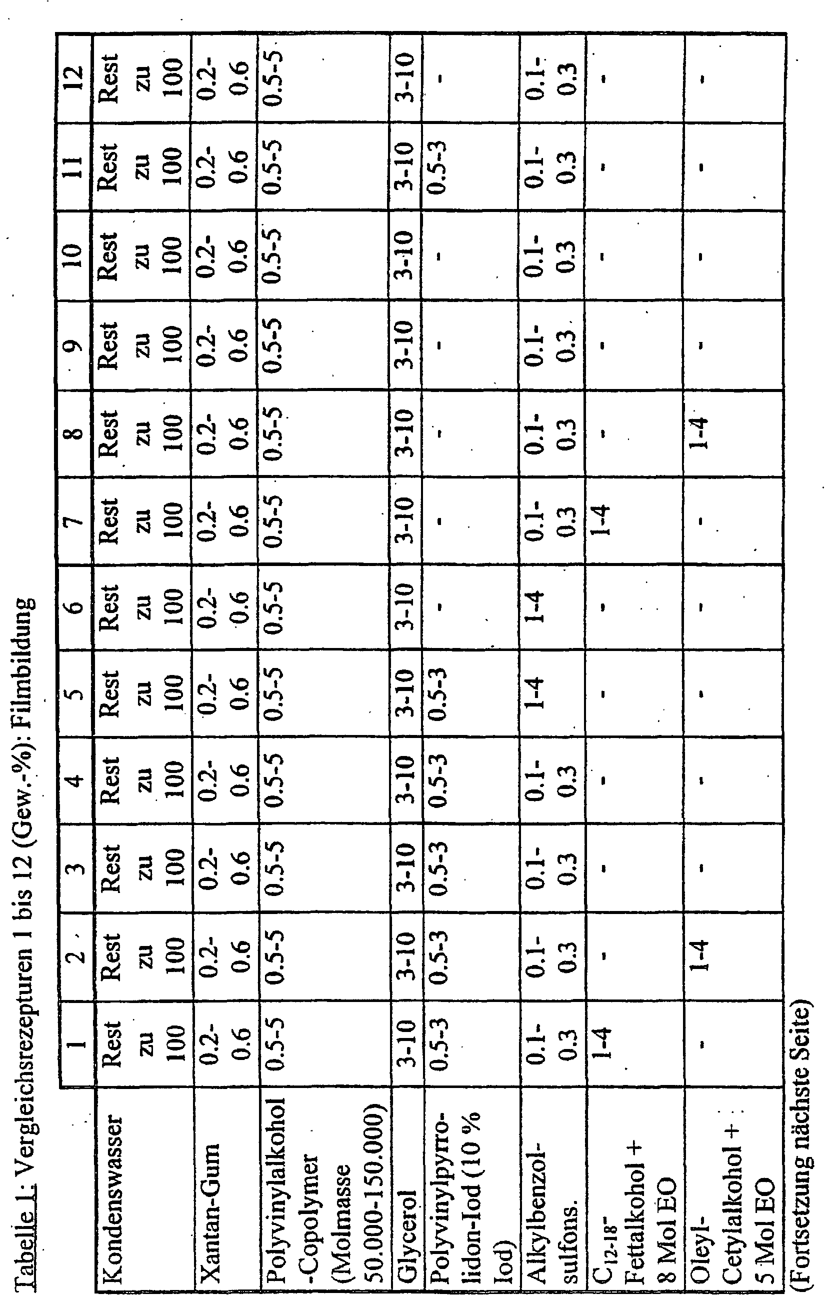

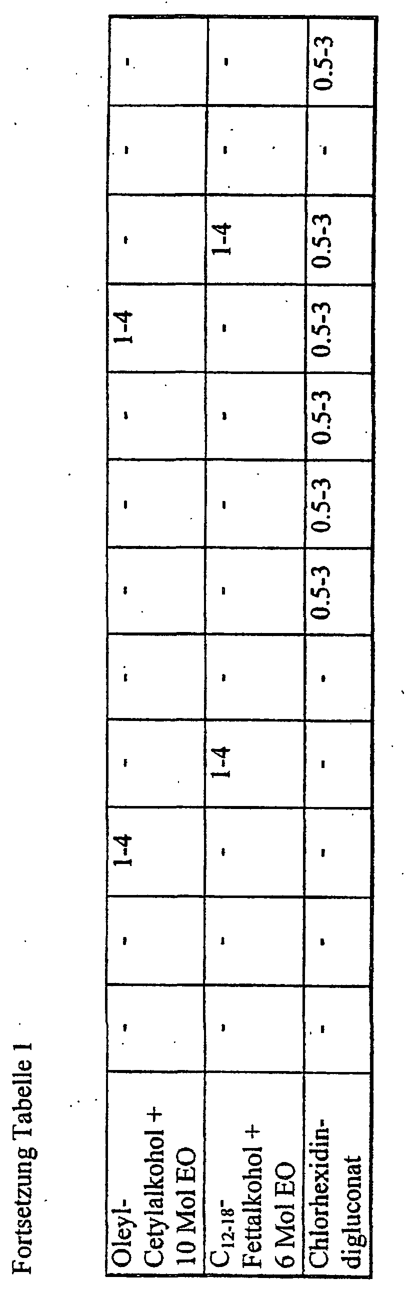

Der Nachteil dieser Rezepturen (Nr. 1 - 12) besteht in der schlechten Filmbildung. Die Kombination von PVP-Iod bzw. Chlorhexidindigluconat mit einem Filmbildner wie PVA ergibt fest, folienartige Filme auf der Zitzenhaut, die sich auch mit Wasser nicht mehr in der zur Verfügung stehenden Zeit entfernen lassen (Rezeptur 11 + 12). Der Zusatz von üblichen, anionaktiven bzw. nichtionogenen Tensiden führt zu klebrigen Rückständen auf der Zitzenhaut. Eine feste, trockene Filmbildung wird hiermit nicht erzielt. Diese klebrigen Filme werden zu früh durch das Liegen der Kühe abgerieben und bieten somit keinen Langzeitschutz auf der Zitze. Ein weiterer Nachteil ist, daß Schmutzrückstände an dem klebrigen Film haften.

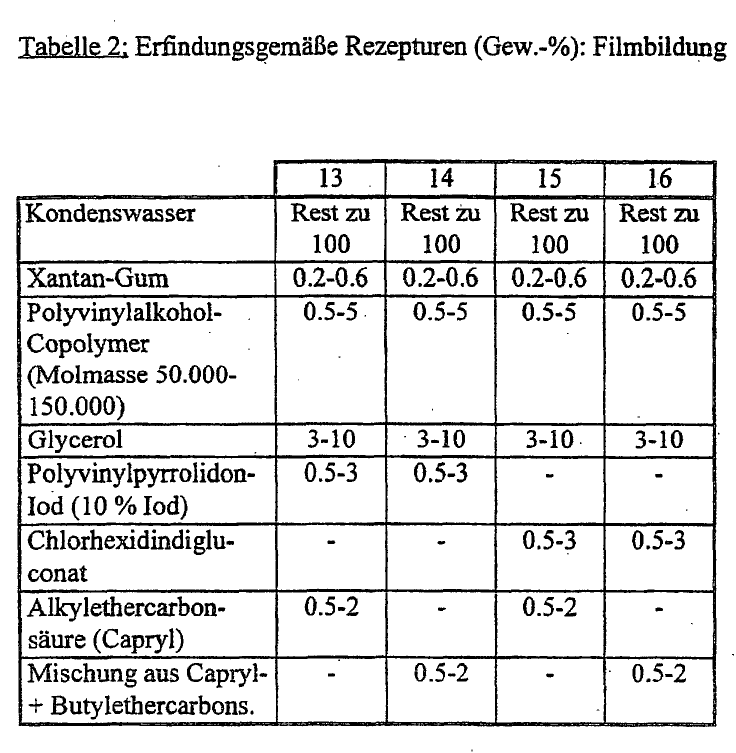

Überraschenderweise wurde gefunden, daß die Verwendung von Ethercarbonsäuren der allgemeinen Formel R(OC₂H₄)ₙOCH₂COOH in Kombination mit PVA als Filmbildner und PVP-Iod bzw. Chlorhexidindigluconat als Desinfektionssubstanz gut haftende, trockene, die Zitzenhaut schützende Filme bildet. Diese Filme lassen sich innerhalb kurzer Zeit (10 - 60 Sekunden) mit Hilfe feuchter Tücher oder unter Verwendung anderer, wäßriger Einweichmittel, sehr leicht von der Zitzenhaut entfernen (Rezeptur 13 - 16).

## Patentansprüche

1. Filmbildendes Mittel zum Schutz vor Infektionen, das eine filmbildende Komponente und einen oder mehrere desinfizierende Wirkstoffe enthält, **dadurch gekennzeichnet, daß** es
a) als filmbildende Komponente 10 bis 100 g/l Polyvinylalkohol-Polymere oder Copolymere mit Molmassen im Bereich von 18.000 bis 200.000,
b) als desinfizierenden Wirkstoff 0,2 bis 3 g/l Iod in Form von Polyvinylpyrrolidon-Iod und/oder 5 bis 50 g/l Chlorhexidin und
c) 2 bis 50 g/l Ethercarbonsäuren der allgemeinen Formel
R-(OC₂H₄)ₙ-OCH₂-COOH enthält, wobei R für einen Alkylrest mit 4 bis 10 C-Atomen und n für eine Zahl im Bereich 2 bis 8 steht.

2. Mittel nach Anspruch 1, **dadurch gekennzeichnet, daß** die Ethercarbonsäure c) 4 bis 8 C-Atome im Alkylrest R aufweist.

3. Mittel nach einem oder beiden der Ansprüche 1 und 2, **dadurch gekennzeichnet, daß** es zusätzlich 1 bis 10 g/l eines Verdickungsmittels enthält.

4. Mittel nach Anspruch 3, **dadurch gekennzeichnet, daß** das Verdickungsmittel ausgewählt ist aus Xanthan-Gum, Carboxymethylcellulose und Alginaten.

5. Mittel nach einem oder mehrem der Ansprüche 1 bis 4, **dadurch gekennzeichnet, daß** es als desinfizierenden Wirkstoff Iod in Form von Polyvinylpyrrolidon-Iod enthält.

6. Mittel nach einem oder mehreren der Ansprüche 1 bis 5, **dadurch gekennzeichnet, daß** es zusätzlich 1 bis 20 g/l eines oder mehrerer Tenside ausgewählt aus anionischen oder nichtionischen Tensiden enthält.

7. Verwendung des Mittels nach einem oder mehreren der Ansprüche 1 bis 6 zur Herstellung eines filmbildenden Mittels zum Schutz vor Infektionen von Zitzen und Eutern von Rindern, Schafen und/oder Ziegen nach dem Melken.

8. Verwendung nach Anspruch 7, **dadurch gekennzeichnet, daß** man die Zitzen oder Euter von Rindern, Schafen oder Ziegen nach dem Melken in das Mittel eintaucht oder sie mit dem Mittel bestreicht oder besprüht.

9. Verwendung nach einem oder beiden der Ansprüche 7 und 8, **dadurch gekennzeichnet, daß** das Mittel nach dem Aufbringen auf Euter oder Zitzen einen sichtbaren, mechanisch festen Film bildet.

## Claims

1. A film-forming preparation for protection against infections which contains a film-forming component and one or more disinfecting agents, **characterized in that** it contains
a) 10 to 100 g/l of polyvinyl alcohol polymers or copolymers with molecular weights in the range from 18,000 to 200,000 as the film-forming component,
b) 0.2 to 3 g/l of iodine in the form of polyvinyl pyrrolidone iodine and/or 5 to 50 g/l of chlorhexidine as the disinfecting agent and
c) 2 to 50 g/l of ether carboxylic acids corresponding to the general formula R-(OC₂H₄)ₙ-OCH₂-COOH, where R is an alkyl group containing 4 to 10 carbon atoms and n is a number of 2 to 8.

2. A film-forming preparation as claimed in claim 1, **characterized in that** the ether carboxylic acid c) contains 4 to 8 carbon atoms in the alkyl group R.

3. A film-forming preparation as claimed in one or both of claims 1 and 2, **characterized in that** it additionally contains 1 to 10 g/l of a thickener.

4. A film-forming preparation as claimed in claim 3, **characterized in that** the thickener is selected from xanthan gum, carboxymethyl cellulose and alginates.

5. A film-forming preparation as claimed in one or more of claims 1 to 4, **characterized in that** it contains iodine in the form of polyvinyl pyrrolidone iodine as the disinfecting agent.

6. A film-forming preparation as claimed in one or more of claims 1 to 5, **characterized in that** it additionally contains 1 to 20 g/l of one or more surfactants selected from anionic or nonionic surfactants.

7. The use of the preparation claimed in one or more of claims 1 to 6 for the production of a film-forming preparation for protecting the teats and udders of cattle, sheep and/or goats against infection after milking.

8. The use claimed in claim 7, **characterized in that** the teats or udders of cattle, sheep or goats are dipped in or coated or sprayed with the preparation after milking.

9. The use claimed in one or both of claims 7 and 8, **characterized in that** the preparation forms a visible and mechanically strong film after application to udders or teats.

## Revendications

1. Agent filmogène pour la protection contre des infections, qui contient un composant filmogène et une ou plusieurs substances actives désinfectantes,
**caractérisé en ce qu'**
il contient
a) comme composant filmogène, de 10 à 100 g/l de polymères ou copolymères d'alcool vinylique ayant des masses moléculaires dans la plage allant de 18 000 à 200 000,
b) comme substance active désinfectante, de 0,2 à 3 g/l d'iode sous forme de polyvinylpyrrolidone-iode et/ou de 5 à 50 g/l de chlorhexidine et
c) de 2 à 50 g/l d'acides éthercarboxyliques de formule générale R-(OC₂H₄)ₙ-OCH₂-COOH, R représentant un radical alkyle ayant de 4 à 10 atomes de carbone et n représentant un nombre dans la plage allant de 2 à 8.

2. Agent selon la revendication 1,
**caractérisé en ce que**
l'acide éthercarboxylique c) comporte de 4 à 8 atomes de carbone dans le radical alkyle R.

3. Agent selon l'une quelconque des revendications 1 et 2 ou les deux,
**caractérisé en ce qu'**
il contient en outre de 1 à 10 g/l d'un épaississant.

4. Agent selon la revendication 3,
**caractérisé en ce que**
l'épaississant est choisi parmi la gomme xanthane, la carboxyméthylcellulose et des alginates.

5. Agent selon une ou plusieurs des revendications 1 à 4,
**caractérisé en ce qu'**
il contient comme substance active désinfectante de l'iode sous forme de polyvinylpyrrolidone-iode,

6. Agent selon une ou plusieurs des revendications 1 à 5,
**caractérisé en ce qu'**
il contient en outre de 1 à 20 g/l d'un ou plusieurs tensioactifs choisis parmi des tensioactifs anioniques et des tensioactifs non ioniques.

7. Utilisation de l'agent selon une ou plusieurs des revendications 1 à 6, pour la fabrication d'un produit filmogène destiné à la protection contre des infections des trayons et des pis de bovins, ovins et caprins après la traite.

8. Utilisation selon la revendication 7,
earactérisée en ce qu'
on trempe dans le produit les trayons ou les pis de bovins, ovins et caprins après la traite ou on les enduit avec le produit ou on pulvérise le produit sur ceux-ci.

9. Utilisation selon l'une quelconque des revendications 7 et 8 ou les deux,
**caractérisée en ce qu'**
après l'application sur les trayons ou les plis, le produit forme un film visible, mécaniquement résistant.
